# EUROPEAN PATENT APPLICATION

(11) **EP 0 951 921 A2**
(43) Date of publication of application: **27.10.1999**
(21) Application number: 99303165.7
(22) Date of filing: 23.04.1999
(51) Int. Cl.: A61N 5/00

(54) **Human tissue energy application system**

(30) Priority: 24.04.1998 US 66103
(71) Applicant: INDIGO MEDICAL, INCORPORATED, Cincinnati Ohio 45242 (US)
(72) Inventor: Ritchie, Paul G., Loveland,, Ohio 45140 (US); Sierocuk, Thomas J., West Chester, Ohio 45069 (US)
(74) Representative: Mercer, Christopher Paul

(57) **Abstract**

The present invention provides an apparatus for applying energy to human tissue, which includes an energy generator and a usage-limited, energy delivery device, and ancillary information exchange capability therebetween for the purposes of security, reliability and operational monitoring and control. The invention also provides a usage-limited, energy delivery device for use in such an apparatus, a method for determining the suitability of the delivery device for use in such an apparatus, and a method of using the delivery device in such an apparatus. The present invention provides an efficient medical treatment system having ready data-communication capability between its permanent and non-permanent components, for the security, reliability and operational monitoring and control desired in such medical systems.

## Description

### FIELD OF THE INVENTION

The present invention relates generally to a system for applying energy to human tissue and, more particularly, to such a system having ancillary information exchange capability. The present invention also relates to a particular component of the system, namely, a usage-limited energy delivery device, and to methods associated therewith, namely, a method for determining the suitability of such a device for use in the system and a method of using such a device in the system.

### BACKGROUND OF THE INVENTION

Key-like electronic information devices and electrical receptacles for use therewith are known. See U.S. Patent No. 4,326,125 to Flies, U.S. Patent No. 4,578,573 to Flies et al., and U.S. Patent No. 4,752,679 to Wehrmacher, the disclosures of which are incorporated herein by this reference. For example, in U.S. Patent No. 4,326,125, Flies discloses a key-like device containing a microelectronic circuit element and having electrical leads exposed along the length of the key for contacting electrical leads in a key receptacle when the key is inserted and turned in the receptacle. The receptacle provides an interface between the microelectronic circuit element of the key and an electronic circuitry system, such as a computer. While Flies concerns himself with improvements in the functional design of such electrical key-like devices and receptacles, he does not disclose incorporation of same in particular systems, such as systems for treating the human body.

Systems which combine permanent and non-permanent devices and provide data communication capability therebetween have been disclosed. Various of these systems concern the treating, analyzing, pumping or infusing of bodily or physiological fluids.

For example, in U.S. Patent No. 4,897,789, King *et al.* disclose an apparatus, including a microprocessor, which is used in the excorporeal photo-activated treatment of body fluids. Disposable devices, including a flat plate irradiation device and a light array assembly, are inserted into the apparatus during use. On each of these devices is mounted an integrated circuit having a non-volatile electronic memory containing encoded data. When the devices are inserted into the apparatus, the integrated circuit becomes electrically connected to the microprocessor, such that decoding of the data can take place. Decoding of the data provides for the authentication and verification of the suitability of the devices for use with the apparatus.

Further by way of example, in U.S. Patent No. 4,975,647, Downer *et al.* disclose a blood analyzer, including a microprocessor, which is adapted to hold a replaceable fluids pack. A fluids identification pod is attached to the fluids pack, such that when the pack is installed in the analyzer, the pod is electrically connected to the microprocessor. Data stored in the pod is thus read by the microprocessor for operations such as the calibration of the analyzer.

Examples of fluid-control systems are disclosed in U.S. Patent No. 5,246,422 to Favre and U.S. Patent No. 5,643,212 to Coutré et *al.* More particularly, Favre discloses a device for pumping fluid to irrigate and to drain biological tissue, wherein the device includes a removable cassette and a permanent module. The removable cassette has a memory device for storing a predetermined pump-control program and the permanent module has means which are actuated by the control program, such that pump operation is effected.

Coutré et *al.* disclose a system for infusing fluids into a patient, which provides a computer-based pharmacy management system which is located remotely with respect to the infusion pumping system, such as in the pharmacy of a hospital. The pharmacist enters information into the computer system to generate a bar code label for a container of the infusion formulation. Once the container makes its way to the health practitioner, he or she enters the data from the label into the infusion pumping system, such that infusion pumping can begin.

There is a need for specific medical treatment systems, combining permanent and non-permanent devices, which provide data communication capability between such devices for the purposes of security, reliability and operational monitoring and control.

### SUMMARY OF THE INVENTION

According to a primary aspect of the present invention, briefly and generally, an apparatus for applying energy to human tissue is provided which includes a means for generating energy and a usage-limited means for delivering energy. The generating means may generate infrared, radio-frequency, or ultrasound energy, or other energy suitable for the particular energy application. The delivering means may be limited to a certain number of energy applications or to a single energy application, and thus, may be designed as a conveniently disposable device. By way of example, the generating means may be a laser and the delivering means may be a disposable fiber optic device. The generating means includes means for connecting with a connection end of the delivering means, for ultimate energy delivery from a distal end of the delivering means to the tissue.

According to the present invention, the generating means includes a means for communicating data, such as a microprocessor. Additionally, the delivering means includes a means for transferring data between it and the generating means, including means for storing and receiving data, such as a memory device. Upon connection of the generating means and the delivering means for energy delivery, the data-communicating means and the data-transferring means are in operable connection for the communication of data. By way of example, the data-transferring means may contain programmed data, such as information concerning usage of the delivering means prior to an energy application, and may accept data from the generating means, such as updated information concerning usage of the delivering means subsequent to the energy application which may be pertinent to the use, or non-use, of the usage-limited delivery device in a future energy application.

According to one aspect of the invention, an energy generator and a delivery device are connected, whereupon a microprocessor within the generator accesses a memory of the delivery device and configures operation of the generator based on identification and parameter information contained in the memory. Should the user desire a different operational configuration, he or she may simply change the contents of the memory or exchange the delivery device for another, as opposed to making direct alterations to the generator itself. During or following use of the delivery device in an energy application, the microprocessor modifies the memory of the delivery device to track and to enforce its usage limitations and to preserve detailed parameters of the energy application for failure analysis, troubleshooting or data collection.

The present invention thus provides an apparatus for applying energy to human tissue, which includes an energy generator and a usage-limited, energy delivery device, and provides ancillary information exchange capability therebetween for the purposes of security, reliability and operational monitoring and control. The present invention also provides a disposable energy delivery device for use in such an apparatus, and methods associated therewith, as further described herein.

Additional objects, advantages and features of the present invention will become apparent from the following description of its preferred embodiments, which description should be taken in conjunction with the accompanying drawings.

### BRIEF DESCRIPTION OF THE DRAWINGS

Figure 1 is an elevational view of an apparatus for applying energy to human tissue which includes an energy generator, an energy delivery device and means for transferring data therebetween, shown in a disconnected configuration, according to an embodiment of the present invention.

Figure 2 is an elevational view of an apparatus for applying energy to human tissue which includes an energy generator, an energy delivery device and means for transferring data therebetween, shown in a disconnected configuration, according to an alternate embodiment of the present invention.

Figure 3 is an elevational view of a modified embodiment of the apparatus of Figure 2, shown in a connected configuration.

Figure 4 is an enlarged cross-sectional view of the energy delivery device and the data transferring means of the apparatus of Figure 1.

Figure 5 is an enlarged cross-sectional view, with internal exposure, of a connecting portion of the apparatus of Figure 1, showing a connecting means of the energy generator and the data transferring means of Figure 4, in a connected configuration.

Figure 6 is an enlarged cross-sectional view of the energy delivery device and the data transferring means of the apparatus of Figure 2.

Figure 7 is an enlarged cross-sectional view of a modified embodiment of the data transferring means of the apparatus of Figure 2.

Figure 8 is an enlarged cross-sectional view, with internal exposure, of a connecting portion of the apparatus of Figure 2, showing connecting means of the energy generator and the data transferring means of Figure 7, in a connected configuration.

Figure 9A is an enlarged cross-sectional view of the electronic circuitry of the data transferring means of the apparatus of Figure 1.

Figure 9B is a side-elevational view of the electronic circuitry of the data transferring means of the apparatus of Figure 1, as viewed along lines 9B shown in Figure 9A.

Figure 10 is a schematic illustration of data communication between an energy generator and a data transferring means, according to the present invention.

Figure 11 is a flow chart illustrating a method for determining the suitability of an energy delivery device for use in an apparatus for applying energy to tissue, according to the present invention.

Figure 12 is a flow chart illustrating a method for using an energy delivery device in an apparatus for applying energy to tissue, according to the present invention.

Figure 13 is a flow chart illustrating a method for writing data to a memory of an energy delivery device, following use of such device in an apparatus for applying energy to tissue, according to the present invention.

### DESCRIPTION OF PREFERRED EMBODIMENTS

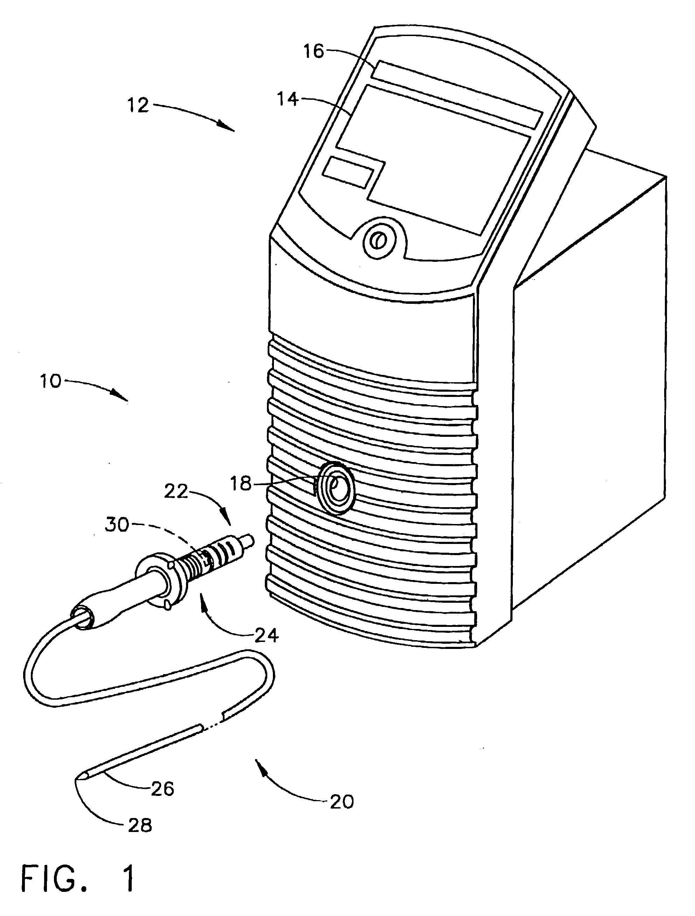

An apparatus 10 for applying energy to human tissue which includes means for generating energy 12 and means for delivering energy 20, is illustrated in a disconnected configuration in Figure 1, according to an embodiment of the present invention.

The means for generating energy 12 may be a generator, such as a laser, capable of generating infrared, radio-frequency, ultrasound or other energy suitable for the treatment of tissue. By way of example, means for generating ultrasonic energy may be the Ultracision Harmonic Scalpel commercially available from Ethicon Endo-Surgery Inc., of Cincinnati, Ohio, and means for generating radio-frequency energy may be any of a variety of surgical generators, such as the ICC 350 Electrosurgical Generator commercially available from Erbe USA, Inc., of Marietta, Georgia. Preferably, the means for generating energy 12 is a laser, and most preferably, the 830e laser system commercially available from Indigo Medical, Incorporated.

The means for delivering energy 20 may be usage-limited, such as a disposable fiber optic device, for delivering energy from an energy source to tissue one time only or for a set number of times. Preferably, the means for delivering energy 20 is the fiberoptic system associated with the 830e laser system, which is also commercially available from Indigo Medical, Incorporated.

In this description of preferred embodiments, "means for generating energy" and "energy generator", "generator" or the like, and "energy delivering means" and "energy delivery device", "delivery device" or the like, respectively, are used interchangeably unless otherwise specified. Additional terms will be used in the same manner, as will be clear to the reader. Further, the terms "distal" and "proximal" are used to refer to relative locations nearest to and farthest from, respectively, the energy delivering end 26 of the apparatus 10, as shown in Figure 1. These conventions are adopted merely by way of convenience, not by way of limitation.

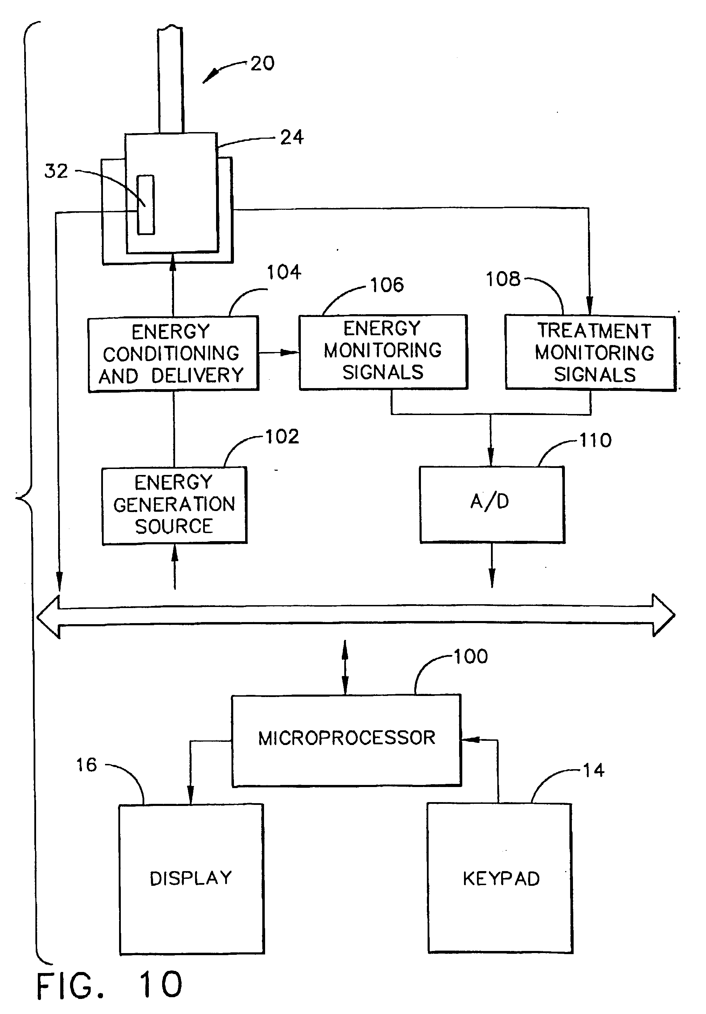

As shown in Figure 1, the energy generator 12 may include a keypad 14 for user interface with a means for communicating data (not shown). The generator 12 may also include a display 16 for the display of data from the data-communicating means. As schematically shown in Figure 10, the data-communicating means may be a microprocessor 100 which is operably connected to the keypad 14 and the display 16.

In operation, the data-communicating means, preferably in the form of microprocessor 100, directs the energy application process according to instructions from the user via the keypad 14 or programmed instructions from the energy delivering device 20, as further described herein. The microprocessor communicates information concerning the process to the display 16 for observation by the user. Should the user find the information concerning the process undesirable, for example, unsafe to the patient undergoing treatment, he or she may override the operating instructions via the keypad 14.

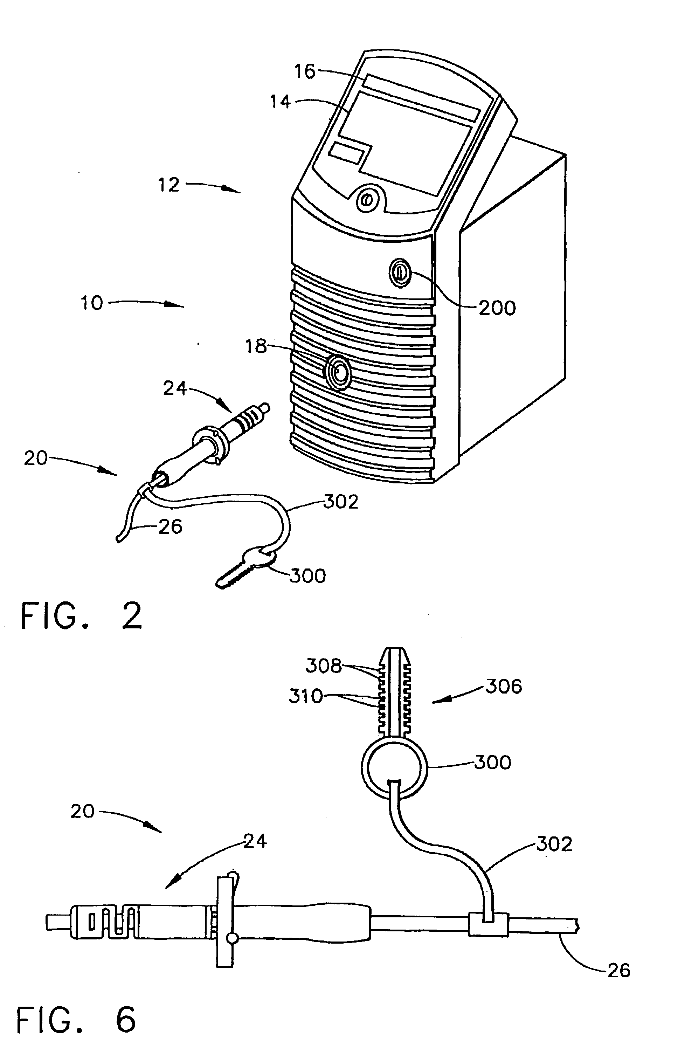

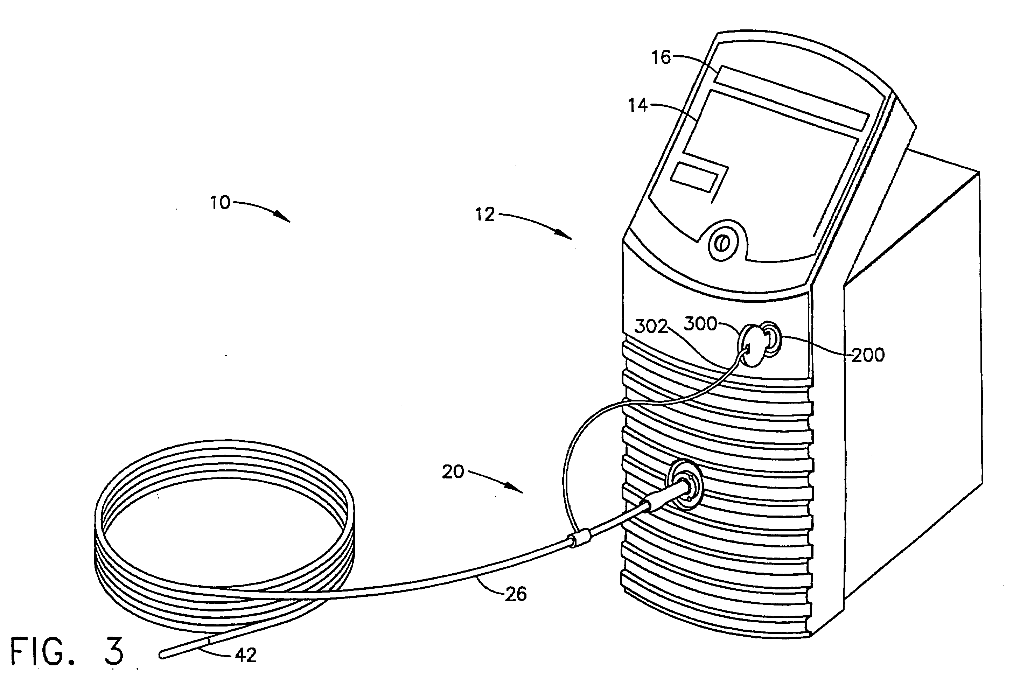

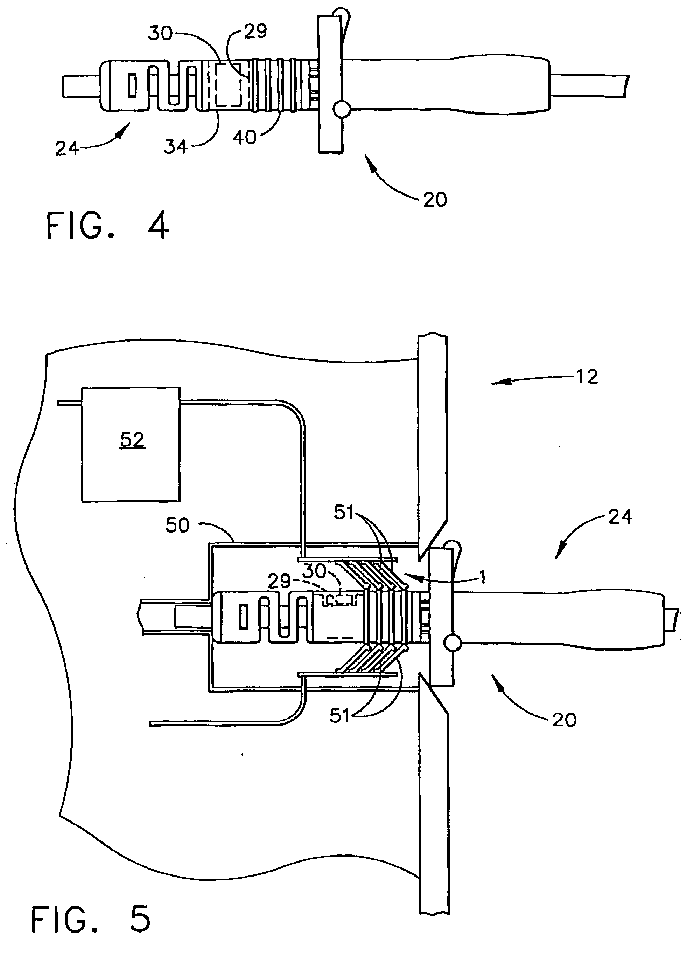

As further shown in Figure 1, the energy generator 12 includes means for connecting 18 with the energy delivery device 20. The connecting means may be any suitable connector of a construction sufficient for connection with the energy delivery device, and may be a female connector, such as the port 18 shown in a disconnected configuration in Figures 1 and 2, and in a connected configuration in Figures 3 and 5.

Complementarily, the energy delivery device 20 has a connection end 24 of a construction sufficient for connection with the connecting means 18 of the generator. The connection end may include any suitable connector of such construction, and may include a male connector, such as the connector 24 shown in a disconnected configuration in Figures 1 and 2, and in a connected configuration in Figures 3 and 5.

Preferably, the connecting means, the connecting end, and the method of using of same, are those disclosed in co-pending United States Patent Application Serial No. 08/915,546, entitled "Fiber Optic Radiation Transmission System, Connector System for an Optical Fiber, and Methods of Using Same" and filed on August 13, 1997 by Evans *et al.* as a continuation of United States Patent Application Serial No. 08/551,009 filed on October 31, 1995, now abandoned. The entire disclosure of this co-pending application is incorporated by this reference.

Upon connection of the delivery device 20 to the generator 12, the delivery device is ready to receive energy from the generator at its proximal end 22 for delivery to the tissue (not shown) from its distal end 26.

In a particularly preferred embodiment, the delivery device 20 includes an optical fiber 26 at its distal end as shown in Figure 1. Preferably, the delivery device 20 includes in a means for diffusing energy from the delivery device to the tissue at its distal end, such as a diffuser 42 in the form of a diffusing tip, as shown in Figure 3. It is preferable that the diffusing means diffuses energy to the tissue in a substantially uniform distribution thereabout, particularly along its length, to avoid producing a hot tip that may damage the tissue being treated.

Preferably, the energy delivery means, the energy diffusing means, and the methods of using same, are those disclosed in co-pending United States Patent Application Serial No. 08/550,846, entitled "Light-Diffusing Device for an Optical Fiber, Methods of Producing and Using Same, and Apparatus for Diffusing Light from an Optical Fiber" and filed on October 31, 1995 by Esch, the entire disclosure of which is incorporated by this reference.

For certain applications, such as interstitial energy application to tissue, the delivery device 20 should be of a construction sufficient for insertion into the tissue. In such cases, the distal end of the delivery device preferably includes a tissue-puncturing tip 28, as shown in Figure 1. Preferably, the tip is composed of a strong material, such as gold or platinum, having a thermal conductivity sufficient to avoid producing a hot tip during the energy application. Preferably, the tip is that disclosed in the above-referenced U.S. Patent Application Serial No. 08/550,846. Further, it is preferable that the system and method for inserting the delivery device into tissue is that disclosed in U.S. Patent No. 5,469,524, entitled "Fiberoptic Delivery System and Method of Use" and issued on November 21, 1995 to Esch *et al.*, the entire disclosure of which is incorporated herein by this reference.

In the embodiment of Figure 1, the energy delivery device includes a means for transferring data 30 between the delivery device and the generator. That is, the data-transferring means 30 is connected to the delivery device, such that when the delivery device is connected to the generator, the data-transferring means becomes operably connected to the data-communicating means (not shown), such as the microprocessor 100 of Figure 10, for the communication of data between the data-transferring means 30 and the data-communicating means. The data-transferring means and the data-communication means may be operably connectable via a variety of means, such as electrical, magnetic, and optical means, or any combination thereof. An example of a suitable operable connection between the data-transferring means 30, shown disposed on the delivery device, and the data-communicating means is described below in relation to Figure 5.

The data-transferring means 30 includes means for storing primary data, such as information indicative of usage (that is, usage and lack of usage) of the delivery device prior to an energy application procedure, and means for receiving secondary data from the data-communicating means, such as information indicative of usage of the delivery device during or following the energy application procedure.

By way of example, in addition to usage-related information just described, the primary data may include information concerning any of the following: identification of the delivering means; expiration, or non-expiration, of the delivering means; parameters for the calibration of the delivering means; the type of energy delivery; operational parameters; energy delivery parameters; monitoring sequence parameters; and any combination thereof, as further described herein. Further by way of example, the secondary data may include information concerning any of the following: identification of the generating means; identification, type, date, or time of treatment; indication or identification of error; amount of energy delivery; integrity of data; and any combination thereof, as further described herein.

The data-transferring means 30 may be any communication device suitable for the above-described data communication. By way of example, the data-transferring means 30 may be a communication device, such as a microprocessor, an integrated memory device, an integrated circuit including an electronically-erasable, programmable, read-only memory ("EEPROM"), and the like.

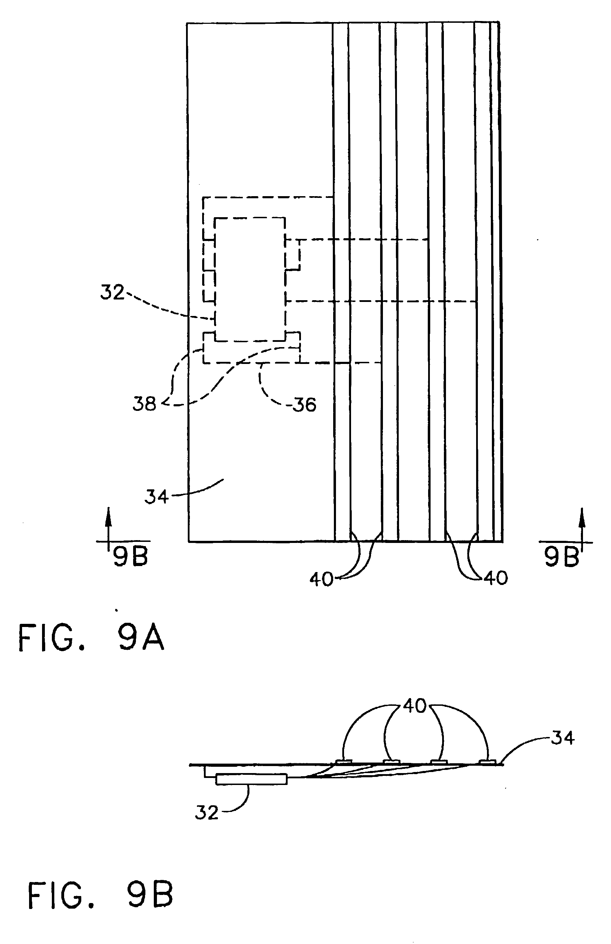

In a preferred embodiment, the data-transferring means is an integrated memory device 30, as shown in Figure 4, which is positioned to make electrical contact upon connection of the delivery device and the generator, as shown in Figure 5. In a particularly preferred embodiment, the memory device includes an EEPROM circuit 32 which is mounted in a housing 34, as shown in Figure 9A. Preferably, the housing is a non-conductive, flexible, supportive housing 34, having flexible leads 36 therein which connect EEPROM pins 38 with one or more conductive pads 40. The non-conductive portion of the flexible housing may be screen-printed, labeled, or otherwise marked to provide for visual identification of the energy delivery device.

The flexible housing 34 allows for cylindrical mounting of the embedded memory device 30 around the external surface of the connection end 24 of the energy delivery device, as shown in Figure 4. In this embodiment, the rigid memory device 30 is disposed over a flattened surface 29, as shown in both Figures 4 and 5. Additionally, the conductive pads 40 are disposed on the exterior surface of the housing 34, as shown in Figures 9B, 4 and 5. When the memory device is cylindrically mounted around the connection end of the energy delivery device 20, as shown in Figure 4, contact between the conductive pads 40 and conducting contacts 51 within the generator 12 is facilitated, as shown in Figure 5.

As further shown in Figure 5, the generator 12 houses a female housing 50, conducting contacts 51, and data-communicating means 52, such as a microprocessor or other generator circuitry. When the energy delivery device 20 is connected to the generator, as shown, the housing 50 accepts the proximal end 22 of the delivery device and the conducting contacts 51 contact the conductive pads 40 to provide an electrical connection between the memory device 30 and the generator circuitry 52. This electrical connection in no way interferes with energy delivery.

Once this electrical connection is established, the generator circuitry 52 is enabled to do any of the following: provide power to the memory device; control the delivery of energy from the generator to the delivery device; receive primary data from the memory device; alter primary data stored in the memory device; provide secondary data to the memory device; and any combination thereof. Additionally, upon receipt of signals from the memory device, such as signals indicative of treatment parameters, the generator circuitry may monitor the energy delivery from the generator to the delivery device. Further, upon receipt of signals from the delivery device, such as signals indicative of a temperature of the tissue, the generator circuitry may monitor delivery of energy to the tissue.

The generator circuitry 52 is shown in greater detail in Figure 10. As previously described in relation to Figure 1, the energy generator may include a keypad 14 and a display 16, both of which are operably connected to the microprocessor 100. The user may interface with the microprocessor by operating the keypad and by reading output from the microprocessor appearing in the display.

As depicted in the block diagram of Figure 10, when the delivery device is connected to the generator, the microprocessor reads the contents of the memory circuit 32, which are preferably in digital form. Based on these memory contents and the energy delivery monitoring signals 106, the microprocessor controls the source of energy generation 102 and the conditioning and delivery of energy 104 from the source to the delivery device 20. More particularly, the microprocessor reads the limits on energy delivery and on returned signals from the memory circuit 32 and monitors energy delivery by reading and analyzing the level of one or more signals, such as a signal 106 coming from the energy conditioning and delivery circuitry 104. Further, the microprocessor monitors tissue treatment by reading and analyzing one or more signals, such as a signal 108 returned from the delivery device 20. By way of example, signal 108 may represent a temperature of the tissue. The analog signals 106 and 108 are converted via an analog-to-digital (A/D) converter 110 to be received by the microprocessor in digital form. In this manner, the microprocessor may access information specific to the energy delivery device and specific to the energy delivery application, and may adjust the energy delivery, treatment, and monitoring sequence based on such information.

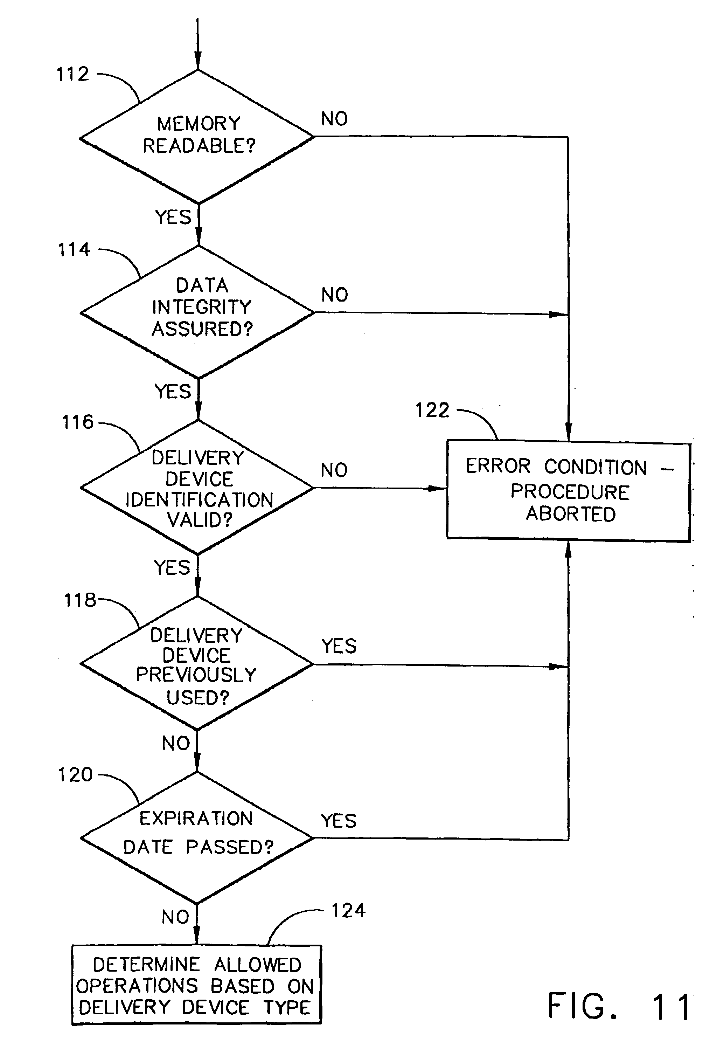

The delivery device 20 is manufactured to facilitate the provision of such specific information to the microprocessor 100. That is, during the manufacture of the delivery device, information specific to that device, such as its identification, expiration date, prior usage, associated type of energy, calibration parameters, and the like, is programmed into the memory circuit 32 of the delivery device. As illustrated by the flow chart of Figure 11, the microprocessor (not shown) reads this programmed information and determines the suitability or unsuitability of the delivery device for use in the energy application apparatus 10. The microprocessor further determines the available options and actions based on the contents of the memory circuit.

By way of example, the microprocessor attempts to read the memory device 112, to assure data integrity 114, to verify the identification information 116, to determine any prior usage 118, to determine whether or not the expiration date has passed 120, and the like. If the memory device cannot be read, the data is compromised, the identification information is incorrect, the delivery device has been used beyond its usage-limitation, the expiration has passed, or any other error occurs, the microprocessor generates an error message 122, which may be displayed via display 16, and aborts the procedure 122. If no error occurs, the microprocessor reads the information, such as the type of optical fiber delivery device and other contents stored in the memory device, to determine the type of energy delivery available 124.

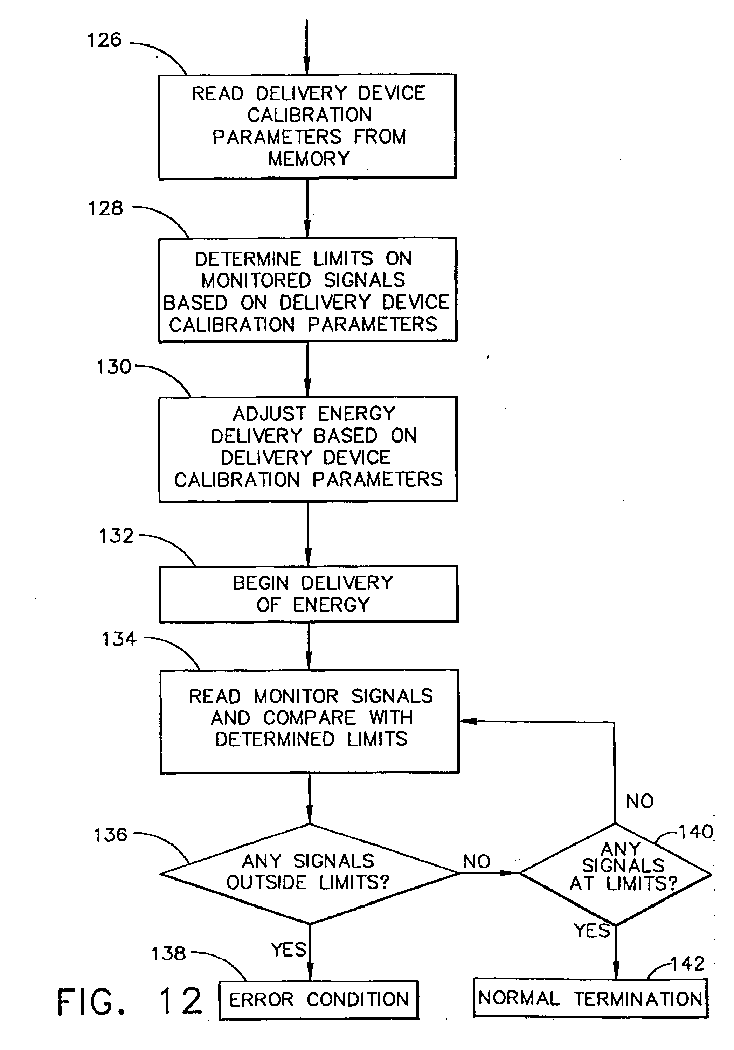

The microprocessor then adjusts the energy delivery based on calibration parameters specific to the delivery device. More particularly, as illustrated by the flow chart of Figure 12, the microprocessor reads the calibration parameters 126 from the memory device and, based on these calibration parameters, determines the limits 128 on the monitored signals, such as signals 106 and 108 discussed above, and adjusts energy delivery 130 accordingly. The microprocessor then allows for energy delivery 132 to the tissue, during which it monitors signals 134, for example, those relating to the energy delivery parameters, such as the amount of energy being delivered, and those relating to the treatment parameters, such as power and temperature.

The microprocessor compares the monitored signals 134 with the limits previously determined 128. If the energy delivery or treatment parameters are outside the limits 136, an error message is generated 138 by the microprocessor and the treatment is aborted, as may be indicated to the user via display 16. If these parameters are not outside the limits 136, energy delivery and monitoring continues until the energy delivery application is normally terminated 140, according to the application limits previously determined, whereupon a termination message is generated 142 by the microprocessor and the treatment is terminated, as may be indicated via display 16.

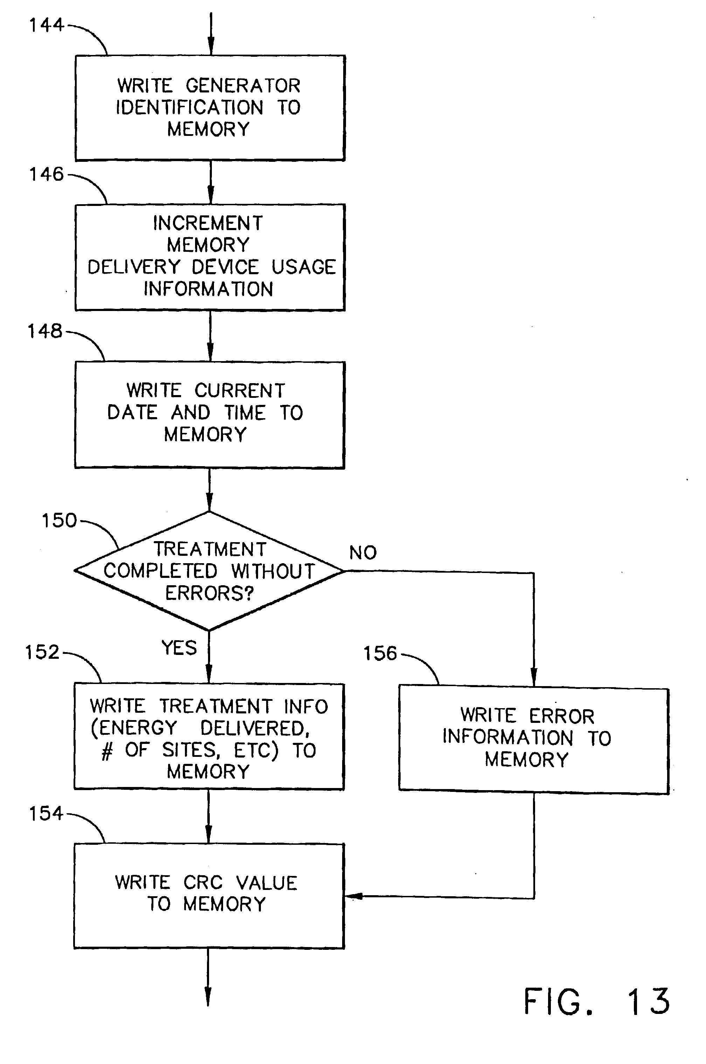

Upon the completion of an energy delivery application or test, the microprocessor alters primary data stored in the memory device or writes modified, secondary data to the memory device, as illustrated in the flow chart of Figure 13. For example, data concerning the type of treatment and generator identification 144 are written to the memory device. Additionally, the usage information 146, the current date and time 148, and the like, are updated and written. If an error occurred 150, error identification and related information are also written 156. By way of example, such error identification and related information may include same associated with tracking and diagnosing field failures. If the treatment was terminated normally, the amount of energy delivered, treatment type, and the like, are written 152. Data integrity information, such as information regarding a checksum or a cyclic redundancy check, are also written 154 to the memory device.

In this manner, the present invention provides an apparatus for applying energy to human tissue, which includes an energy generator and a usage-limited, energy delivery device, and ancillary information exchange capability therebetween for the purposes of security, reliability and operational monitoring and control. The invention also provides a usage-limited, energy delivery device for use in such an apparatus, a method for determining the suitability of the delivery device for use in such an apparatus, and a method of using the delivery device in such an apparatus.

Aspects of the preferred embodiment described above are representative of the present invention. It will be understood that various modifications and alternatives are contemplated and fall within the scope of the invention. Merely by way of example, while the memory device has been described as possibly including an EEPROM, which may store a significant amount of data (such as 16 kilobytes), it may alternatively include any of a variety of integrated circuits providing memory capability. Further, while the memory device has been described as possibly taking the form of an externally mounted, flexible assembly, it may alternatively be integrated into the delivery device in other forms or configurations, or via inset molding or other methods. Additionally, while the ancillary information exchange between the delivery device and the generator has been described as possibly being accomplished via electrical means, it may alternatively be accomplished via magnetic or optical means. These alternatives and others which may be arrived at by one of ordinary skill in the art without undue experimentation, are contemplated as being within the scope of the present invention.

An alternative embodiment of the present invention is now described in relation to Figures 2, 3 and 6-8. For convenience, reference numbers used to refer to features of the previously described embodiment are used to refer to similar features of this alternative embodiment.

Figure 2 shows the apparatus 10, which includes the generator 12 and the energy delivery device 20, as previously described. According to this embodiment, the generator includes a key receptacle 200 and the energy delivery device has a key means 300 attached thereto, such as by a flexible tether 302 or a less flexible attachment means 304 such as that shown in Figure 3.

The key means may be used for a variety of purposes, such as for turning the generator on or off and for transmitting and receiving information specific to the delivery device, as previously described. Further, the key means may include a bar-code device which is optically scanned for information, an electronic circuit board including an EEPROM device which is capable of being electrically coupled to the generator through the key receptacle, an EEPROM device disposed on or in the key means which is capable of being electrically coupled to the generator through the key receptacle, or a microprocessor which is capable of being electrically coupled to the generator through the key receptacle.

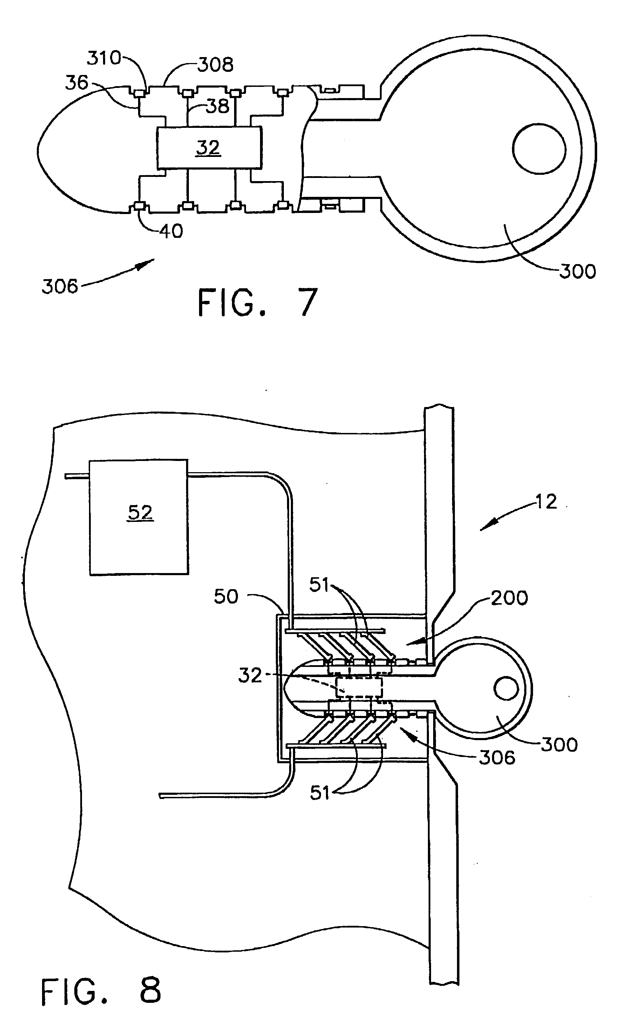

By way of example, as shown in Figure 6, the key means 300 may include means 306 for interacting with the key receptacle. Such interacting means 306 may take the form of a series of protrusions 308 and recessions 310, as shown, or other suitable forms. When the key means 300 is manipulated in the key receptacle 200, as shown in Figure 3, the data-transferring means (not shown) of the delivery device becomes operably connected to the data-communicating means (not shown) of the generator. It may be necessary to manipulate the key means 300 in the key receptacle 200 beyond mere insertion, such as by rotation of the key means or other suitable methods, to effect such operable connection.

According to this alternative embodiment, and as shown in Figures 2 and 6, no memory device appears on the connection end 24 of the delivery device. That is, the memory device is located on the key means 300, as opposed to the delivery device itself.

The details of the key means 300 are shown in Figure 7. The key means includes an EEPROM circuit 32, one or more conductive pads 40, and leads 36 for connecting the EEPROM pins 38 with the conductive pads. As shown in Figure 8, the generator 12 houses the key receptacle 200, as well as the female housing 50, conducting contacts 51, and data-communicating means 52, such as a microprocessor or other generator circuitry, as previously described in relation to Figure 5. When the key means 300 is inserted in the key receptacle 200, as shown, the housing 50 accepts the interacting means 306 of the key and the conducting contacts 51 contact the conductive pads 40 to provide an electrical connection between the EEPROM 32 and the generator circuitry 52. Once this connection is established, the apparatus may be operated as previously described in relation to the preferred embodiment.

While the present invention has been described in terms of a data-transferring means, a communication device, a key means, or a key, which is either connected to or attached to (i.e., disposed on, or tethered to) an energy-delivering means or an energy-delivery device, the present invention merely requires that the former is uniquely associated with the energy-delivery means or energy delivery device. That is, the former must be identifiable with the latter and contain information specific to the latter to be "associated with" the latter, within the meaning of the present invention. By way of example, it is contemplated that the former may be delivered to the user in the same package with the latter, and have pre-programmed information specific to the latter, and so be "associated with" the latter, within the meaning of the present invention, without having to be connected to or attached to the latter, as shown in the above-described embodiments.

As described herein, the present invention provides, *inter alia,* an apparatus for applying energy to human tissue, which includes an energy generator and a usage-limited, energy delivery device, and ancillary information exchange capability therebetween for the purposes of security, reliability and operational monitoring and control. Such an apparatus may be used in a variety of medical procedures, such as the treatment of benign prostate hypertrophy, as disclosed in the above-referenced patents and applications of Esch and Esch et *al.,* which have been incorporated herein by reference. The present invention thus provides an efficient medical treatment system having ready data-communication capability between its permanent and non-permanent components, for the security, reliability and operational monitoring and control desired in such medical systems. The invention also provides a usage-limited, energy delivery device for use in such an apparatus, a method for determining the suitability of the delivery device for use in such an apparatus, and a method of using the delivery device in such an apparatus.

Although the various aspects of the present invention have been described with respect to the preferred embodiments thereof, it will be understood that the invention is entitled to protection within the full scope of the appended claims.

## Claims

1. An apparatus for applying energy to human tissue, comprising:
(a) means for generating energy, said generating means comprising: (i) means for connecting with means for delivering energy and (ii) means for communicating data;
(b) usage-limited means for delivering energy, said delivering means comprising: (i) a connection end of a construction sufficient for connection with the connecting means of said generating means to provide for energy delivery from said generating means to said delivering means and (ii) a distal end for delivery of energy to the tissue; and
(c) means for transferring data between said delivering means and said generating means, said transferring means being associated with said delivering means and operably connectable to the communicating means of said generating means for communicating data therebetween, said transferring means comprising means for storing primary data and for receiving secondary data from the communicating means of said generating means, said primary data including information indicative of usage of said delivering means prior to an energy application and said secondary data including information indicative of usage of said delivering means during or following the energy application.

2. The apparatus of claim 1 wherein the energy is infrared, radio-frequency or ultrasound energy.

3. The apparatus of claim 1 or claim 2 wherein said generating means is a laser.

4. The apparatus of any one of claims 1 to 3 wherein, upon connection of said delivering means to said generating means, electrical contact is established therebetween.

5. The apparatus of any one of claims 1 to 4 wherein the communicating means of said generating means controls the delivery of energy from said generating means to said delivering means.

6. The apparatus of any one of claims 1 to 5 wherein, upon connection of said delivering means to said generating means, the communicating means of said generating means provides power to said transferring means.

7. The apparatus of any one of claims 1 to 6 wherein, upon connection of said delivering means to said generating means, the communicating means of said generating means receives the primary data from said transferring means.

8. The apparatus of any one of claims 1 to 7 wherein, upon connection of said delivering means to said generating means, the communicating means of said generating means alters the primary data stored in said transferring means.

9. The apparatus of any one of claims 1 to 8 wherein, upon connection of said delivering means to said generating means, the communicating means of said generating means provides the secondary data to said transferring means.

10. The apparatus of any one of claims 1 to 9 wherein the communicating means of said generating means comprises a microprocessor.

11. The apparatus of any one of claims 1 to 10 wherein the communicating means of said generating means monitors the energy delivery from said generating means to said delivering means via signals from said transferring means.

12. The apparatus of any one of claims 1 to 11 wherein the communicating means monitors the delivery of energy to the tissue via signals from said delivering means.

13. The apparatus of any one of claims 1 to 12 wherein said delivering means comprises an optical fiber at the distal end.

14. The apparatus of any one of claims 1 to 13 wherein said delivering means comprises means for diffusing energy from the delivering means to the tissue, the diffusing means being disposed at the distal end of said delivering means.

15. The apparatus of claim 14 wherein the diffusing means comprises a diffusing tip.

16. The apparatus of claim 14 or claim 15 wherein the diffusing means diffuses energy to the tissue in a substantially uniform distribution about the diffusing means.

17. The apparatus of any one of claims 1 to 16 wherein the distal end of said delivering means is of a construction sufficient for insertion into the tissue.

18. The apparatus of claim 17 wherein the distal end of said delivering means comprises a tissue-puncturing tip.

19. The apparatus of claim 18 wherein the tip is composed of a material having a thermal conductivity sufficient to avoid producing a hot tip during energy delivery to the tissue.

20. The apparatus of any one of claims 1 to 19 wherein said delivering means is limited to one usage.

21. The apparatus of any one of claims 1 to 20 wherein said transferring means is disposed on said delivering means such that, upon connection of said delivering means to said generating means, said transferring means is operably connected to the communicating means of said generating means.

22. The apparatus of any one of claims 1 to 21 wherein said transferring means and said generating means are operably connectable for communicating data therebetween via electrical, magnetic or optical means or any combination thereof.

23. The apparatus of any one of claims 1 to 22 wherein said transferring means comprises a microprocessor.

24. The apparatus of any one of claims 1 to 22 wherein said transferring means comprises an integrated memory device.

25. The apparatus of claim 24 wherein the memory device comprises an EEPROM integrated circuit.

26. The apparatus of any one of claims 1 to 25 wherein said generating means further comprises a key receptacle and said transferring means comprises a key means associated with said delivering means, wherein upon manipulation of the key means in the key receptacle, said transferring means is operably connected to the communicating means of said generating means.

27. The apparatus of claim 26 wherein the key means comprises a circuit board including an integrated memory device for electrical coupling with the key receptacle.

28. The apparatus of claim 26 wherein the key means includes an integrated memory device and electical contacts for electrical coupling with the key receptacle.

29. The apparatus of any one of claims 1 to 28 wherein the primary data further includes information on delivering means identification, delivering means expiry, delivering means usage, delivering means calibration parameters, type of energy for delivery, operational parameters, energy delivery parameters or monitoring sequence parameters or any combination thereof.

30. The apparatus of any one of claims 1 to 29 wherein the secondary data includes information on generating means identification, treatment identification, date of treatment, time of treatment, error indication, error identification, amount of energy delivery, type of treatment or data integrity or any combination thereof.

31. A disposable device for delivering energy from a source of energy to human tissue, wherein the energy source includes means for communicating data, said device comprising:
means for delivering energy, said delivering means having a connection end of a construction sufficient for connection with the energy source to provide for energy delivery from the energy source to said delivering means and having a distal end for delivery of energy to the tissue; and
means for transferring data between said delivering means and the energy source, said transferring means being associated with said delivering means and operably connectable to the communicating means of the energy source for communicating data therebetween, said transferring means including means for storing primary data and for receiving secondary data from the communicating means of the energy source, said primary data including information indicative of usage of said delivering means prior to an energy delivery procedure and said secondary data including information indicative of usage of said delivering means during or following the energy delivery procedure.
